# EUROPEAN PATENT SPECIFICATION

(11) **EP 1 771 463 B1**
(45) Date of publication and mention of the grant of the patent: **02.01.2008**
(21) Application number: 05770278.9
(22) Date of filing: 13.07.2005
(51) Int. Cl.: C07K 1/00, C07C 231/02, C07B 43/06

(54) **METHOD OF PEPTIDE SYNTHESIS**
VERFAHREN FÜR PEPTIDSYNTHESE
METHODE DE SYNTHESE DE PEPTIDES

(30) Priority: 16.07.2004 EP 04016776; 20.07.2004 EP 04017037; 20.07.2004 EP 04017036
(43) Date of publication of application: 11.04.2007
(73) Proprietor: Lonza AG, 4052 Basel (CH)
(72) Inventor: GIRAUD, Matthieu, CH-1950 Sion (CH); WILLINER, Michaela, CH-3902 Glis (CH); WERBITZKY, Oleg, CH-3968 Veyras (CH)
(74) Representative: Riegler, Norbert Hermann
(86) International application number: PCT/EP2005/007625
(87) International publication number: WO 2006/008050

(56) References cited:
- US-A1- 2002 193 594
- US-A1- 2003 060 459
- US-A1- 2003 219 854

## Description

The present invention relates to the field of pharmaceutically useful polypeptides, namely to a respective method of chemical derivatization enhancing biological activity of such peptides. It devises a method of chemically amidating the C-terminus of a polypeptide.

Small peptide drugs have gained importance as a distinct, useful class of pharmaceutically active ingredients. For instance, anti-infective peptides, particularly cationic peptides whose industrial biotechnological manufacture is devised in US 2003/0219854 A1, are a new class of broad spectrum antimicrobial substances which may help to combat the rapid spread of multi-drug resistance towards standard antibiotics amongst pathogenic microbes.

Naturally occurring anti-infective peptides include post-translational modifications such as C-terminal amidation that are paramount for sustained biological activity (Boman, Immunol. Rev. 173:5, 2000). For instance, C-terminal amidation eliminates potential charge and further protects from rapid degradation by ubiquitous exopeptidases. Whereas fully chemical synthesis may suitably introduce C-terminal amide functions during synthesis (e.g. Han et al., J. Org. Chem. 1996, 61, 6326-6339; Albericio, F. and Barany, G. , Int. J. Peptide Protein Res. 30, 1987, 206-216), the more cost-effective biotechnological route processing single peptide from large head-to-tail peptide concatemers lacks this possibility. Hence a biotechnologically manufactured peptide according to US 2003/0219854 A1 must be C-terminally amidated in a downstream processing step. The chemical amidation approaches described so far entailed a considerable degree of racemisation of the peptide backbone though.

US 2003/0219854 describes terminal amidation of a tryptophan rich, Boc-protected 12-mer by reaction with an excess of ammonia and a base reagent (20 eq) in aprotic solvent, DMF, in the presence of near stoichiometric amounts (6 eq) of an uronium coupling reagent (HATU: O-(7-azabenzotriazol-1-yl)-1,1,3,3-tetramethyluronium hexafluorophosphate) and of the co-activator N-hydroxy-9-azabenzotriazole. The alleged yield of the amidated, deprotected peptide derivative was 47% .

Careful reproduction of this reaction sequence and reaction product analysis (s. exp. section) shows that the low yield is mainly due to unwanted epimerisation of the C-terminal amino acid, giving rise to two amidated diastereomers in almost 1:1 ratio as confirmed by electropherograms obtained with both a Tween 20 and a Cyclodextrin CE method. This ratio obtained was also confirmed by a reverse-phase LC/ESI-MS tandem chromatography using Marfey's reagent as a chiral derivatizing agent of the hydrolyzed peptide as described in detail in the experimental section of the present application. That is half of the product is lost upon amidation due to efficient racemisation of the C-terminal amino acid.

Another method applicable only in the context of solid-phase synthesis is described in Albericio, F. and Barany, G. , Int. J. Peptide Protein Res. 30,1987,206-216. The method is however intricate in requiring preparation of special FMOC-trialkoxy-benzylamide handles for functionalisation of the resin support. After coupling and synthesis of the peptide to the support, cleavage from the thus modified resin support yields readily amidated peptide at about 80% optical purity of the desired diastereoisomer, which adds to a lower cleavage efficiency to give a total yield only of about 60 to 70% of the desired amidated diastereoisomer of the peptide. A similar approach with chemically slightly different amide handles is described by the same author in US 5306562.

The object of the present invention is to avoid the disadvantages of the prior art and to devise another or improved method of chemical, non-enzymatic amidation of a non-protected α-carboxyl group of an peptide or amino acid. According to the present invention, this object is solved by a method for amidating the free α-carboxyl group of an amino acid or peptide, comprising a first step of reacting said amino acid or preferably said peptide with a peptide coupling reagent in an organic solvent in the presence of a base and further in the presence of an ammonium salt of at least one peptide coupling additive wherein the ammonium cation is selected from the group consisting of protonated ammonia, protonated primary amine and protonated secondary amine, and wherein the side chain and α-amino function or said amino acid or peptide are protected with non-base-labile protecting groups.

It is to be understood that the base reagent and the ammonium cation of the salt preferably are not identical and hence are not forming a conjugated acid-base pair. Rather, that there is a first base and a second base which second base is the ammonium cation according to the present invention. In the following, the term 'base' shall always be construed as to refer to said first base only. The second base will always be referred to as the ammonium cation.

The method of the present invention has the advantage of minimizing adverse epimerisation of the α-carbon atom of the amino acid residue that is actually amidated at its α-carboxyl group. In di- or higher n-mer peptide, this amino acid residue is the C-terminal residue of a peptide which is a particularly preferred embodiment of the present invention.

The peptide or polypeptide according to the present invention may be any peptide. The protection of side chains and α-amino function will allow of achieving a sufficient solubility of a suitably protected peptide or amino acid in the organic solvent. It goes without saying that the solvent conditions according to the present invention prove favorably for efficient amidation under retention of configuration, but may prove denaturing in view of leaving secondary or even tertiary structure of longer peptides unharmed, especially when such secondary or tertiary structure is not stabilized by covalent intrachain bonds or similar, possibly non-natural structural elements. Preferably, a peptide according to the present invention comprises 100 or less amino acid residues, more preferably 50 or less amino acid residues, most preferably 20 or less amino acid residues. This definition includes peptides comprising non-natural composites such as D-amino acids, L- or D- amino acids with modified or unusual side chains or non-amino-acid building blocks linking different parts of the peptide chain within afore said peptide. Solid-phase synthesis allows of efficient synthesis of peptides close to 50 residues, though further liquid-phase segment condensation reaction allows of producing even more lengthy fully synthetic peptides. Now length restriction exists for peptides obtained from biotechnological production. Reduction of the water content of the reaction mixture, as well as suitably excluding other protic solvents, in particular excluding besides water lower C1-C5 alkyl alcohols such as methanol, isopropanol or ethanol, is preferred for the present invention.
Preferably, the peptide is an antimicrobial or anti-infective, bactericidal peptide, preferably is a cationic antimicrobial peptide such as e.g. ILRWPWWPWRRK, namely indolicidin, which are more active in their C-terminally amidated form. Anti-infective peptides, particularly cationic peptides such as indolicidin derivatives and in particular ILRWPWWPWRRK whose industrial biotechnological manufacture is devised in US 2003/0219854 A1, are a new class of broad spectrum antimicrobial substances which may help to combat the rapid spread of multi-drug resistance towards standard antibiotics amongst pathogenic microbes.

Preferably the amidation reaction is carried out having a water content of less than 25% (v/v), more preferably less than 15% (v/v), most preferably less than 5% (v/v). In a further preferred embodiment, the reaction is carried out under essentially water-free conditions. This may include using e.g. freshly distilled solvent or protective nitrogen atmosphere.

Preferably the organic solvent is an aprotic organic solvent, more preferably it is a polar aprotic organic solvent. Suitable examples are acetonitrile, dimethyl sulfoxide, dichloromethane and N,N-dimethylformamide. Most preferably the organic aprotic solvent is N,N-dimethylformide.

Preferably the amidation reaction is carried out at -15°C to 50°C and the pH is controlled during reaction to be in the range of about 8 to 9, preferably is controlled at about 8.5.

The type of reagents referred to above such as coupling agents, coupling additives and protecting groups are well-known from standard peptide synthesis, which in essence is an amidation reaction, and are described in detail e.g. in Bodanszky, M. , Principles of Peptide Synthesis, 2nd ed., Springer Verlag Berlin/Heidelberg, 1993).

Non-base-labile protecting groups are well-known in the art, the term "base-labile" being construed as to refer to abstraction at basic pH and/or aminolysis by primary or secondary amines as is common in the art, Suitable examples are e.g. 2-nitro-methoxyphenylsulfenyl-, Alloc (allyloxycarbonyl)-, Z (benzyloxycarbonyl)-, Boo (tert-butoxycarbonyl)-, Bpoc-(biphenylyl-isopropoxycarbonyl)-group. Orthogonal protection schemes including base labile protection are of course excluded from the present invention. Whether global protection with a single type of protecting group or protection by different types of protecting groups in the same peptide or amino acid is required, depends on the source of the peptide from chemical or biotechnological synthesis and the type of side chains comprised in the peptide or amino acid. Notably, removal of such non-base-labile groups may be effected by different means or under different reaction conditions. For instance, the Nps (o-nitrophenylsulfenyl) group is favorably removed by sulfhydryl nucleophils, Z-groups are removed by hydrogenolysis or Alloc groups may be removed by Pd(I)-catalysed hydrogenation. According to the present invention, suitably non-base labile protection groups are preferably removed after amidation by acidolysis, as e.g. feasible for Boc, Z, trityl, Nps or Bpoc groups.

From the gist of the present invention, it is apparent that protection of aspartyl and glutamyl side chains that might be present in the peptide according to the present invention deserves special attention and requires suitable carboxyl protecting groups for selective protection or masking of ω-carboxyl groups leaving the C-terminal α-carboxyl group. This can be achieved, for instance, by global esterification of carboxyl-groups with a benzyl halogenide to yield a benzyl ester, followed by regioselective cleavage of the α-ester with LiOH in acetone (Bryant, P. et al., 1959, J. Chem. Soc., p. 3868 ff.) or by selective esterification of ω-carboxyl groups with alkyl halogenides in the presence of Cu(II) salts masking the α-function during esterification by complex formation (Ledger, R., 1965, Austral. J. Chem. 18:1477ff); deprotection of the ω-carboxyl group may also be facilitated by Cu(II) catalysis (Prestidge, R., 1975, J. Org. Chem. 40:3287ff.).

In another preferred embodiment of the present invention, the peptide or amino acid to be amidated is free from carboxyl groups other than the C-terminal α-carboxyl group to be amidated, preferably is free from glutamyl or aspartyl residues.

Given that the generation of glutaminyl or asparaginyl residues by concomitant ω-carboxyl groups is desireable and has been taken into account in the overall synthetic strategy of a peptide or amino acid according to the present invention, it is a further independent object of said invention to devise a method for amidating the free α-carboxyl group of an amino acid or peptide, comprising the step of reacting said amino acid or peptide with a peptide coupling reagent in the presence of a first base and further in the presence of an ammonium salt of at least one peptide coupling additive wherein the ammonium cation is selected from the group consisting of protonated ammonia, protonated primary amine and protonated secondary amine and wherein the side chain and α-amino function of said amino acid or peptide are protected with non-base-labile protecting groups with the exception of the ω-carboxyl groups of individual aspartyl or glutamyl residues and wherein said ω-carboxyl groups are amidated concomitantly with the free α-carboxyl group in the reaction. This second object is particularly preferred when using ammonia for the salt compound, yielding natural amino acid amides, namely asparaginyl and/or glutaminyl.

All technical explanations and descriptions of preferred embodiments of the invention given above or in the following sections which relate to the first object of the invention extend likewise to this second object unless apparently incompatible with such second object of the present invention, e.g. when claiming the complete absence of any aspartyl or glutamyl residue in the peptide.

Coupling reagents for peptide synthesis are well-known in the art (see e.g. Bodanszky, supra). Coupling reagents may be mixed anhydrides (e.g. T3P: propanephosphonic anhydride) or other acylating agents such as activated esters or acid halogenides (e.g. ICBF, isobutyl chloroformate), or they may be carbodiimides, activated benzotriazine derivatives (DEPBT: 3-(diethoxyphosphoryloxy)-1,2,3-benzotriazine-4(3H)-one) or uronium or phosphonium salt derivatives of benzotriazole. In one preferred embodiment of the present invention, the coupling reagent is a coupling reagent other than a carbodiimide.

In a preferred embodiment, the coupling reagent is selected from the group consisting of uronium salts and phosphonium salts which have been found to give best total yields and best protection against racemization in the method of the present invention.

Further, more preferred is that the coupling reagent is selected from the group consisting of uronium salts and phosphonium salts of the benzotriazole capable of activating said α-carboxyl group and that the reaction is carried out in the presence of a base. Suitable and likewise preferred examples of such uronium or phosphonium coupling salts are e.g. HBTU (O-(1H-benzotriazol-1-yl)-N,N,N',N'-tetramethyluronium hexafluorophosphate), BOP (benzotriazol-1-yloxy-tris(dimethylamino)phosphonium hexafluorophosphate), PyBOP (benzotriazol-1-yloxy-tripyrrolidinophosphonium hexafluorophosphate), PyAOP, HCTU (O-(1H-6-chlorobenzotriazol-1-yl)-1,1,3,3-tetramethyluronium hexafluorophosphate), TCTU (O-(1H-6-chlorobenzotriazol-1-yl)1,1,3,3-tetramethyluronium tetrafluoroborate), HATU (O-(7-azabenzotriazol-1-yl)-1,1,3,3-tetramethyluronium hexafluorophosphate), TATU (O-(7-azabenzotriazol-1-yl)-1,1,3,3-tetramethyluronium tetrafluoroborate), TOTU (O-[cyano(ethoxycarbonyl)methyleneamino]-N,N',N',N'-tetramethyluronium tetrafluoroborate), HAPyU (O-(benzotriazol-1-yl)oxydipyrrolidinouronium hexafluorophosphate.

Preferably, the base or first base is a weak base whose conjugated acid has a pKa value of from pKa 7.5 to 15, more preferably of from pKa 7.5 to 10, with the exclusion of an α-amino function of a peptide or amino acid or amino acid derivative, and which base preferably is a tertiary, sterically hindered amine. Examples of such and further preferred are Hünig base (N,N-diisopropylethylamine), N,N-dialkylaniline, 2,4,6-trialkylpyridine or N-alkylmorpholine with the alkyl being straight or branched C1-C4 alkyl, more preferably it is N-methylmorpholine or collidine (2,4,6-trimethylpyridine), most preferably it is collidine. All preferred embodiments described above and below are particulary preferred being worked in combination with a first weak base reagent as described in this section.

In another preferred embodiment, the amidation method is carried out with a carbodiimide as the coupling reagent and particularly is carried out in the presence of a tertiary amine. More preferably, the carbodiimide coupling reagent is selected from the group consisting of diisopropylcarbodiimide, dicyclohexylcarbodiimide and 1-ethyl-3-(3-dimethylaminopropyl)carbodiimide, most preferably is 1 ethyl-3-(3-dimethylaminopropyl)carbodiimide. Carbodiimides are for technical reasons less suited for industrial upscaling of synthetic processes, though. Preferably, the reaction using a carbodiimide as the coupling reagent is carried out in the presence of a second coupling additive other than said ammonium salt which second additive is a protonated, i.e. non-ionic N-hydroxybenzotriazole or N-hydroxybenzotriazole derivative compliant with the below given definitions.

The use of coupling additives, in particular of coupling additives of the benzotriazole type, is also known. Hence it is further preferred that the coupling reagent additive is a nucleophilic hydroxy compound capable of forming activated esters, more preferably having an acidic, nucleophilic N-hydroxy function wherein N is imide or is N-acyl or N-aryl substituted triazeno, most preferably the coupling additive is a N-hydroxy-benzotriazole derivative (1-hydroxy-benzotriazole derivative) or is an N-hydroxybenzotriazine derivative. Such coupling additive N-hydroxy compounds have been described in WO 94/07910 and EP-410 182. Examples are e.g. N-hydroxysuccinimide, N-hydroxy-3,4-dihydro-4-oxo-1,2,3-benzotriazine, 1-hydroxy-7-azabenzotriazole and N-hydroxybenzotriazole. N-Hydroxybenzotriazine derivatives are particularly preferred, in a most preferred embodiment the coupling reagent additive is hydroxy-3,4-dihydro-4-oxo-1,2,3-benzotriazine.
Ammonium salt compounds of coupling additives are known and have been described, for instance in US 4806641.

In a further particularly preferred embodiment, the uronium or phosphonium salt coupling reagent is an uronium salt reagent and preferably is HCTU, TCTU or HBTU and even more preferably is used in the reaction in combination with an ammonium salt of N-hydroxy-3,4-dihydro-4-oxo-1,2,3-benzotriazine.

In the context of the present invention, it is to be noted that HCTU and TCTU are defined as to be encompassed by the term 'uronium salt reagent' despite that these compounds and its analogues of formula I have been shown by means of crystal structure analysis to comprise an isonitroso moiety rather than an uronium moiety (O. Marder, Y. Shvo, and F. Albericio HCTU and TCTU: New Coupling Reagents: Development and Industrial Applications, Poster, Presentation Gordon Conference February 2002), an N-amidino substituent on the heterocyclic core giving rise to a guanidium structure instead. Hence such class of compounds according to formula I is termed guanidium-type subclass of uronium salt reagents according to the present invention: 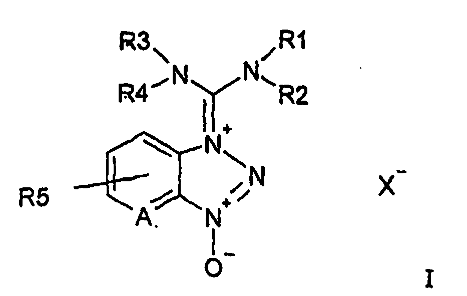 wherein R1, R2, R3, R4 each are alkyl, preferably are independently ethyl or methyl; A is N or C; R5 is H or, preferably, is an electron-withdrawing substitutent, more preferably is chloro; and X is a complex anion, preferably is hexafluorophosphate or tetrafluoroborate.

As for the ammonium cation to be used in the salts of the present invention, the ammonium cation is H₂N⁺R1R2 with R1, R2 independently being H or C1-C10, preferably C1-C5, aliphatic or alicyclic hydrocarbyl that may optionally further be substituted with aryl, alkoxy, aralkoxy, alkylaryl, aryloxy, hydroxy or halogen, preferably excluding N-hetemaromatic moieties. Preferably, R1 and R2 are not further substituted and are alkyl as defined above. More preferably, R1 is H and R2 is methyl, ethyl, propyl or isopropyl or R1 and R2 are both H, i.e. the cation is NH₄⁺ which is most preferred.

Preferably, in a second step after the first reaction step, the amidated amino acid or peptide is isolate and in a third step is deprotected to yield the free side chains and α-amino function. Preferably, the amidated amino acid or peptide is partly or entirely deprotected by acidolysis. In one possible though less preferred embodiment of the present invention, an amino acid is amidated in this way and the pure L-amino acid carboxamide is obtained, suitably retaining side chain masking or protecting groups, for use of such amino acid amide in excess in a normal segment condensation peptide synthesis scheme with a second peptide fragment. In this way, the more expensive peptide as arising e.g. from solid phase synthesis is spared from unnecessary losses due to amidation-caused racemization whilst the more cheaply available amino acid amide may be used in large excess for efficient coupling. Such embodiment is particularly suited for use of ammonia or lower alkyl amines as described above in the amidation reaction according to the method of the present invention.

In a further preferred embodiment, the protected peptide of the present invention is coupled to a conventional resin support by way of side chain anchoring to the functional linker moiety of the resin. Hence the C-terminus is left free for the purpose of amidation. Such support may be any resin commonly used in the art such CTC, Wang or Merrifield resin. In this way, the method of the present invention allows of efficient recovery and deprotection of the amidation product.
Further preferred is that in a second step after the first reaction step, the amidated, protected amino acid or preferably peptide is isolated and in a subsequent third step is deprotected to yield the free side chains and α-amino function, preferably it is partly or entirely deprotected by acidolysis.

In a further embodiment of the present invention, a method of amidating e.g. a biotechnologically produced, unprotected peptide having a free α-carboxyl group is devised, comprising the steps of
a) recovering and isolating said peptide from a concatemeric fusion protein of said peptide, which fusion protein may further comprise interspersed linker sequences,
b) derivatizing at least the α-amino function of the unprotected peptide with non-base-labile protecting groups, preferably also protecting nucleophilic groups of individual amino acid side chains by non-base-labile protecting groups,
c) preferably recovering the protected peptide by precipitation with water,
d) amidating the C-terminal carboxyl group by the method described in the preceding sections,
e) preferably isolating the amidated peptide,
f) and deprotecting the side chains and α-amino function.

All the above preferred embodiments drawn to peptides of the present invention likewise specifically and preferably apply to (C-terminally) amidating antimicrobial peptides, preferably to antimicrobial peptides, most preferably to ILRWPWWPWRRK and/or indolicidin derivatives.

### Examples

### Boc-protection of the 12mer ILRWPWWPWRRK-OH

Protection of the peptide was carried out essentially as described in US 2003/0219854 A1, except for the fact that the product was recovered by precipitation instead of lyophilization. The di-Boc-protected ILRWPWWPWRRK-OH (diBoc-MBI 1187) (ESI-MS: m/z 1980) was used in the subsequent amidation without further purification.
Bocylation was carried out using di-tert-butyl dicarbonate (Boc₂O) in acetonitrile/1 N NaOH/H₂O. The reaction mixture was stirred at room temperature, the pH was then adjusted to pH 4.6 and the organic solvents were removed under vacuum. The pH was then again adjusted to 2. Water was added and the suspension cooled down to 4 °C before isolation of the precipitate thus formed. The solid was washed in water and then dried in vacuo.

### C-terminal amidation of Boc-ILRWPWWPWRRK(Boc)-OH

HCTU (209 mg, 0.5 mmol) is added to a solution of *diBoc*-MBI 11B7 (900 mg, 0.455 mmol), N-methylmorpholine (150 µl, 1.365 mmol) and NH₃/HOOBt (250 mg, 1.365 mmol) in DMF (36ml) and stirred at 25°C under nitrogen atmosphere. If required, the pH is adjusted with N-methylmorpholine to pH 8.5 (±0.5). The reaction mixture is stirred at 25°C for 2h and controlled to maintain a pH of 8.5 (±0.2).
DMF is then evaporated under reduced pressure (water bath temperature <50°C). Water is added (90 ml) and the suspension kept at 4°C for at least 2 h before isolation. The solid is filtered and the cake washed with water (twice 25 ml). The crude peptide is dried under vacuum for 15 h (temperature <50°C). A white powder is obtained (910 mg) that is subjected to further analysis.

Analytical method: Derivatization of single amino acids after complete hydrolysis of peptide

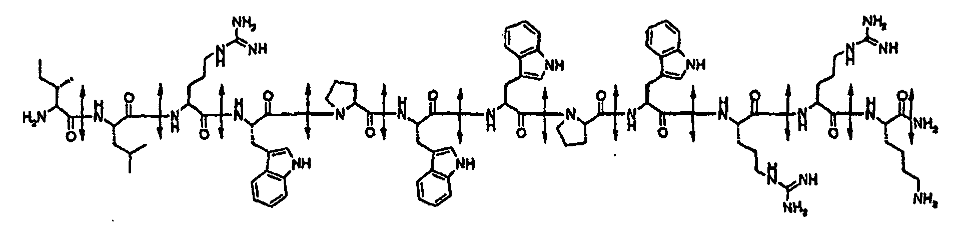

Using a 6N HCl treatment the peptide is hydrolyzed into the single amino acids. During this treatment the C-terminal lysinamide is converted into the corresponding carboxylic acid (Lys-OH) as described by Marfey et al. (Marfey, P., Carlsberg Res. Commun., 49, (1984), 591). Except possibly for Cys, such complete hydrolysis does not promote racemization of amino acids. In short, the method devises to derivatize optical isomers of amino acids in the hydrolysate with FDAA (1-fluoro-2,4-dinitrophenyl-5-L-alanine amide). The simple derivatization procedure is completed within 90 minutes.

In the present context, the lysine derivatives can be easily separated and quantified by reverse phase HPLC. Derivatives have an absorption coefficient of ~3 x 10⁴ and can be detected by UV at 340 nm with picomole sensitivity. First the L-Lys-NH₂ and the D-Lys-NH₂ were hydrolyzed and derivatized. - Conversion rate and hence total yield (conv. %) was determined by HPLC separation of the amidated diastereomers of the educt. The product retaining L-configuration at the terminal lysinamide was identified by the method of Marfey et al., and the relative excess of the non-epimerized L-Lys product vs. the D-Lys product (%P) was determined by HPLC. An HPLC method was developed for the diastereoisomer analysis essentially as described in Marfey et al. and a good separation was obtained.

For the amidation of the same peptide, different combinations of coupling reagents and additives have been tested (Table I). For comparative purposes, where indicated, exceptionally aqueous ammonia along with separate coupling additive was used instead of the ammonium salt of the coupling additive devised according to the present invention (Table I). 'SM' indicates the amount of Boc-ILRWPWWPWRRK(Boc)-OH reacted.

**Table I**

| SM [mg] | C. Rgt. | Co-activ. | Base | Solvent | "NH₃" | Conv.% | %P |
|---|---|---|---|---|---|---|---|
| 26.0 | T3P | - | NMM | DMF | NH₃/HOOBt | 80 | 92.0 |
| 51.0 | HCTU | - | NMM | DMF | NH₃/HOOBt | 100 | 92.5 |
| 51.2 | HBTU | - | NMM | DMF | NH₃/HOOBt | 93 | 93.7 |
| 51.0 | EDC | Cl-HOBt | TMP | DMF | NH₃/HOOBt/CuCl₂ | 79 | 90.3 |
| 51.0 | EDC | Cl-HOBt | TMP | DMF | NH₃/HOOBt | 98 | 95.2 |
| 50.0 | IBCF | - | NMM | DMF | NH₃/HOOBt | 82 | 80.1 |
| 50.4 | IBCF | - | TMP | DMF | NH₃/HOSu | 88 | 82.2 |
| 50.9 | TCTU | - | NMM | DMF | NH₃/HOOBt | 89 | 94.2 |
| *50.1* | *IBCF* | *-* | *NMM* | *DMF* | *Aqueous NH₃* | *85* | *52.6* |
| 304.0 | HCTU | - | NMM | DMF | NH₃/HOOBt | 94 | 94.3 |
| 49.8 | HCTU | - | NMM | DMF. | NH₃/HOOBt | 100 | 94.8 |

The use of an ammonium salt of an co-activating reagent enhances the retention of the C-terminal amino acid configuration.

### Deprotection of Boc-ILRWPWWPWRRK(Boc)-NH₂

Deprotection of 100 mg diBoc-peptides amide takes place in 0.8 ml DMF to which 0.2 mℓ trifluoroacetic acid were added. The mixture is stirred for 30 min. at room temperature. The unprotected peptide-amide is then further purified by reverse phase HPLC and is finally lyophilized (ESI-MS: m/z 1779).

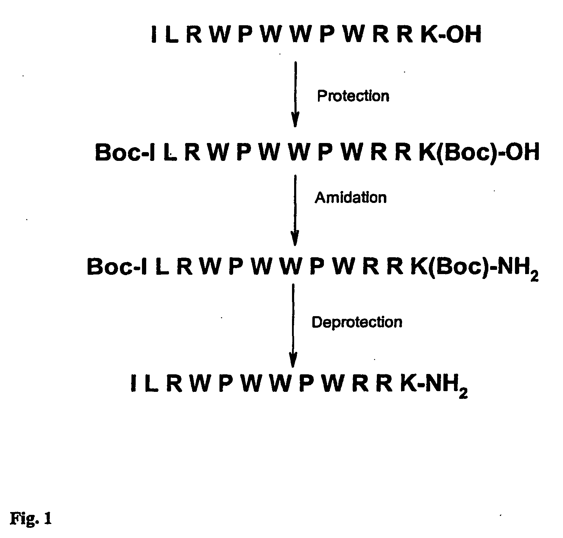

## Claims

1. Method for amidating the free α-carboxyl group of an amino acid or peptide, comprising the first step of reacting said amino acid or peptide, with a peptide coupling reagent in an aprotic organic solvent in the presence of a first base, with the exclusion of an α-amino function of a peptide or amino acid, and in the presence of an ammonium salt of at least one peptide coupling additive, wherein the ammonium cation is selected from the group consisting of protonated of ammonia, protonated primary amine and protonated secondary amine, wherein the pH is controlled during reaction to be in the range of 8 to 9, and wherein the side chain and α-amino function of said amino acid or peptide are protected with non-base-labile protecting groups.

2. Method for amidating the free α-carboxyl group of an amino acid or peptide, comprising the step of reacting said amino acid or peptide with a peptide coupling reagent in an aprotic organic solvent in the presence of a first base, with the exclusion of an α-amino function of a peptide or amino acid, and in the presence of an ammonium salt of at least one peptide coupling additive, wherein the ammonium cation is selected from the group consisting of protonated ammonia, protonated primary amine and protonated secondary amine, wherein the pH is controlled during reaction to be in the range of 8 to 9, and wherein the side chain and α-amino function of said amino acid or peptide are protected with non-base-labile protecting groups with the exception of the ω-carboxyl groups of individual aspartyl or glutamyl residues, and wherein said ω-carboxyl groups are amidated concomitantly with the free α-carboxyl group in the reaction.

3. Method according to claim 1 or 2, **characterized in that** the first base is a weak base whose conjugated acid has a pKa value of 7.5 to 15, preferably of 7.5 to 10.

4. Method according to claim 3, **characterized in that** the weak base is N,N-diisopropylethylamine, 2,4,6-trialkylpyridine or N-alkylmorpholine, preferably with the alkyl being straight or branched C1-C4 alkyl.

5. Method according to one of the preceding claims, **characterized in that** the coupling reagent is selected from the group consisting of uronium salts and phosphonium salts of the benzotriazole capable of activating said α-carboxyl group.

6. Method according to one of the preceding claims, **characterized in that** the organic solvent is a polar aprotic organic solvent.

7. Method according to claim 6, **characterized in that** the solvent is selected from the group consisting of acetonitrile, dimethyl sulfoxide, dimethylacetamide, dichloxomethane and N,N-dimethylformamide, preferably it is N,N-dimethylformamide.

8. Method according to one of the preceding claims, **characterized in that** the coupling reagent additive is a nucleophilic N-hydroxy compound capable of forming activated esters.

9. Method according to claim 8, **characterized in that** the coupling reagent additive is selected from the group consisting of N-hydroxysuccinimide, N-hydroxy-3,4-dihydro-4-oxo-1,2,3-benzotriazine, 1-hydroxy-7-azabenzotriazole and N-hydroxy-benzotriazole.

10. Method according to claim 9, **characterized in that** the coupling reagent additive is hydroxy-3,4-dihydro-4-oxo-1,2,3-benzotriazine.

11. Method according to one of the preceding claims, **characterized in that** the pH is controlled during reaction to be in the range of 8 to 9, preferably is controlled at about 8.5.

12. Method according to claims 1 or 2, **characterized in that** the method is carried out with a carbodiimide as the coupling reagent and, preferably, in the presence of a protonated N-hydroxy benzotriazole as second coupling additive.

13. Method according to claim 5, **characterized in that** the coupling uronium or phosphonium salt reagent is an uronium salt reagent and preferably is HCTU, TCTU or HBTU and more preferably is used in the reaction in combination with an ammonium salt of N-bydroxy-3,4-dihydro-4..oxo-l.2.3-be.nzotriazine.

14. Method according to one of the preceding claims, **characterized in that** the ammonium cation is H₂N⁺R1R2 with R1, R2 independently being H or C1-C10, preferably C1-C5, aliphatic or alicyclic hydrocarbyl.

15. Method according to claim 14, **characterized in that** R1 is H and R2 is methyl, ethyl, propyl or isopropyl; or **in that** R1 and R2 are H.

16. Method according to one of the preceding claims, **characterized in that** in a second step after the first reaction step, the amidated, protected amino acid or peptide is isolated and in a third step is deprotected to yield the free side chains and α-amino function, preferably is partly or entirely deprotected by acidolysis.

17. Method for amidating the tree α-carboxyl group of a peptide ILRWPWWPWRRK, comprising the first step of reacting said ILRWPWWPWRRK with a peptide coupling reagent in an organic solvent in the presence of a first base, with the exclusion of an α-amino function of a peptide or amino acid, and further in the presence of an ammonium salt of at least one peptide coupling additive, wherein the ammonium cation is selected from the group consisting of protonated ammonia, protonated primary amine and protonated secondary amine,, wherein the pH is controlled during reaction to be in the range of 8 to 9, and wherein the side chain and α-amino function of said ILRWPWWPWRRK are protected with non-base-labile protecting groups.

18. Method for amidating the free α-carboxyl group of an antimicrobial peptide; preferably a cationic antimicrobial peptide, comprising the first step of reacting said peptide with a peptide coupling reagent in an aprotic organic solvent in the presence of a first base, with the exclusion of an α-amino function of a peptide or amino acid, and further in the presence of an ammonium salt of at least one peptide coupling additive, wherein the ammonium cation is selected from the group consisting of protonated ammonia, protonated primary amine and protonated secondary amine, wherein the pH is ; controlled during reaction to be in the range of 8 to 9, and wherein the side chain and α-amino function of said peptide are protected with non-base-labile protecting groups.

19. Method according to one of claims 17 or 18, **characterized in that** in a second step after the first reaction step, the amidated, protected ILRWPWWPWRRK or anti-microbial peptide, preferably anti-microbial cationic peptide is isolated and in a third step is deprotected to yield the free side chains and α-amino function, preferably is partly or entirely deprotected by acidolysis.

## Patentansprüche

1. Verfahren zur Amidierung der freien α-Carboxygruppe einer Aminosäure oder eines Peptids, enthaltend als ersten Schritt die Reaktion der besagten Aminosäure oder des besagten Peptids mit einem Peptidkupplungsreagenz in einem aprotischen organischen Lösungsmittel in Anwesenheit einer ersten Base, die nicht eine α-Aminofunktion eines Peptids oder einer Aminosäure ist, und in Anwesenheit eines Ammoniumsalzes mindestens eines Peptidkupplungszusatzes, wobei das Ammoniumkation ausgewählt ist aus der Gruppe bestehend aus protoniertem Ammoniak, protoniertem primären Amin und protoniertem sekundären Amin, wobei während der Reaktion der pH auf einen Wert im Bereich von 8 bis 9 kontrolliert wird, und wobei die Seitenkette und die α-Aminofunktion der besagten Aminosäure oder des besagten Peptids mit nicht-baselabilen Schutzgruppen geschützt sind.

2. Verfahren zur Amidierung der freien α-Carboxygruppe einer Aminosäure oder eines Peptids enthaltend als Schritt die Reaktion der besagten Aminosäure oder des besagten Peptids mit einem Peptidkupplungsreagenz in einem aprotischen organischen Lösungsmittel in Anwesenheit einer ersten Base, die nicht eine α-Aminofunktion eines Peptids oder einer Aminosäure ist, und in Anwesenheit eines Ammoniumsalzes mindestens eines Peptidkupplungszusatzes, wobei das Ammoniumkation ausgewählt ist aus der Gruppe bestehend aus protoniertem Ammoniak, protoniertem primären Amin und protoniertem sekundären Amin, wobei während der Reaktion der pH auf einen Wert im Bereich von 8 bis 9 kontrolliert wird, und wobei die Seitenkette und die α-Aminofunktion der besagten Aminosäure oder des besagten Peptids mit nicht-baselabilen Schutzgruppen geschützt sind ausser der ω-Carboxygruppen von einzelnen Aspartyl- oder Glutamylresten, und wobei besagte ω-Carboxygruppen gleichzeitig mit der freien α-Carboxygruppe in der Reaktion amidiert werden.

3. Verfahren nach Anspruch 1 oder 2, **dadurch gekennzeichnet, dass** die erste Base eine schwache Base ist, deren konjugierte Säure einen pKa-Wert von 7,5 bis 15, bevorzugt von 7,5 bis 10, hat.

4. Verfahren nach Anspruch 3, **dadurch gekennzeichnet, dass** die schwache Base *N,N-*Diisopropylethylamin, 2,4,6-Trialkylpyridin oder *N*-Alkylmorpholin ist, wobei das Alkyl vorzugsweise gerades oder verzweigtes C₁-C₄ Alkyl ist.

5. Verfahren nach einem der vorhergehenden Ansprüche, **dadurch gekennzeichnet, dass** das Kupplungsreagenz ausgewählt ist aus der Gruppe bestehend aus Uroniumsalzen und Phosphoniumsalzen des Benzotriazols und imstande ist, besagte α-Carboxygruppe zu aktivieren.

6. Verfahren nach einem der vorhergehenden Ansprüche, **dadurch gekennzeichnet, dass** das organische Lösungsmittel ein polares aprotisches organisches Lösungsmittel ist.

7. Verfahren nach Anspruch 6, **dadurch gekennzeichnet, dass** das Lösungsmittel ausgewählt ist aus der Gruppe bestehend aus Acetonitril, Dimethylsulfoxid, Dimethylacetamid, Dichlormethan und *N,N*-Dimethylformamid; bevorzugt ist es *N,N*-Dimethylformamid.

8. Verfahren nach einem der vorhergehenden Ansprüche, **dadurch gekennzeichnet, dass** der Kupplungszusatz eine nukleophile *N*-Hydroxyverbindung ist, welche imstande ist, aktivierte Ester zu bilden.

9. Verfahren nach Anspruch 8, **dadurch gekennzeichnet, dass** der Kupplungsreagenzzusatz ausgewählt ist aus der Gruppe bestehend aus *N*-Hydroxysuccinimid, *N-*Hydroxy-3,4-dihydro-4-oxo-1,2,3-benzotriazin, 1-Hydroxy-7-azabenzotriazol und *N*-Hydroxybenzotriazol.

10. Verfahren nach Anspruch 9, **dadurch gekennzeichnet, dass** der Kupplungsreagenzzusatz Hydroxy-3,4-dihydro-4-oxo-1,2,3-benzotriazin ist.

11. Verfahren nach einem der vorhergehenden Ansprüche, **dadurch gekennzeichnet, dass** während der Reaktion der pH auf einen Wert im Bereich von 8 bis 9, bevorzugt auf einen Wert von ungefähr 8,5, kontrolliert wird.

12. Verfahren nach Anspruch 1 oder 2, **dadurch gekennzeichnet, dass** das Verfahren mit einem Carbodiimid als Kupplungsreagenz, und bevorzugt in Anwesenheit eines protonierten *N*-Hydroxybenzotriazols als zweiter Kupplungszusatz, ausgeführt wird.

13. Verfahren nach Anspruch 5, **dadurch gekennzeichnet, dass** das Kupplungsuroniumsalz oder -phosphoniumsalz ein Uroniumsalzreagenz, bevorzugt HCTU, TCTU oder HBTU ist, und besonders bevorzugt in Kombination mit einem Ammoniumsalz des *N*-Hydroxy-3,4-dihydro-4-oxo-1,2,3-benzotriazins in der Reaktion verwendet wird.

14. Verfahren nach einem der vorhergehenden Ansprüche, **dadurch gekennzeichnet, dass** das Ammoniumkation H₂N⁺R¹R² ist, wobei R¹ und R² jeweils unabhängig H oder ein aliphatischer oder alizyklischer C₁-C₁₀, bevorzugt ein C₁-C₅, Kohlenwasserstoff sind.

15. Verfahren nach Anspruch 14, **dadurch gekennzeichnet, dass** R¹ H ist und R² Methyl, Ethyl, Propyl oder Isopropyl ist, oder dass R¹ und R² beide H sind.

16. Verfahren nach einem der vorhergehenden Ansprüche, **dadurch gekennzeichnet, dass** in einem zweiten Schritt nach dem ersten Reaktionsschritt die amidierte, geschützte Aminosäure oder das amidierte, geschützte Peptid isoliert und in einem dritten Schritt entschützt wird, um die freien Seitenketten und α-Aminofunktion zu liefern, wobei es bevorzugt teilweise oder komplett durch Azidolyse entschützt wird.

17. Verfahren zur Amidierung der freien α-Carboxygruppe eines Peptids ILRWPWWPWRRK, enthaltend als ersten Schritt die Reaktion des besagten ILRWPWWPWRRK mit einem Peptidkupplungsreagenz in einem aprotischen organischen Lösungsmittel in Anwesenheit einer ersten Base, die nicht eine α-Aminofunktion eines Peptids oder einer Aminosäure ist, und ferner in Anwesenheit eines Ammoniumsalzes mindestens eines Peptidkupplungszusatzes, wobei das Ammoniumkation ausgewählt ist aus der Gruppe bestehend aus protoniertem Ammoniak, protoniertem primären Amin und protoniertem sekundären Amin, wobei während der Reaktion der pH auf einen Wert im Bereich von 8 bis 9 kontrolliert wird, und wobei die Seitenkette und α-Aminofunktion des besagten ILRWPWWPWRRK mit nicht-baselabilen Schutzgruppen geschützt sind.

18. Verfahren zur Amidierung der freien α-Carboxygruppe eines antimikrobiellen Peptids, bevorzugt eines kationischen, antimikrobiellen Peptids, enthaltend als ersten Schritt die Reaktion des besagten Peptids mit einem Peptidkupplungsreagenz in einem aprotischen organischen Lösungsmittel in Anwesenheit einer ersten Base, die nicht eine α-Aminofunktion eines Peptids oder einer Aminosäure ist, und ferner in Anwesenheit eines Ammoniumsalzes mindestens eines Peptidkupplungszusatzes, wobei das Ammoniumkation ausgewählt ist aus der Gruppe bestehend aus Ammoniak, primäres Amin oder sekundäres Amin, wobei während der Reaktion der pH auf einen Wert im Bereich von 8 bis 9 kontrolliert wird, und wobei die Seitenkette und α-Aminofunktion des besagten Peptids mit nicht-baselabilen Schutzgruppen geschützt sind.

19. Verfahren nach einem der Ansprüche 17 oder 18, **dadurch gekennzeichnet, dass** in einem zweiten Schritt nach dem ersten Reaktionschritt das amidierte, geschützte ILRWPWWPWRRK oder das antimikrobielle, vorzugsweise antimikrobielle kationische, Peptid isoliert und in einem dritten Schritt entschützt wird, um die freien Seitenketten und α-Aminofunktion zu liefern, wobei es vorzugsweise teilweise oder komplett durch Azidolyse entschützt wird.

## Revendications

1. Méthode d'amidation du groupement α-carboxy libre d'un acide aminé ou d'un peptide, qui comprend la première étape de réaction dudit acide aminé ou peptide avec un réactif de couplage peptidique dans un solvant organique aprotique en présence d'une première base, à l'exclusion d'une fonction α-amino d'un peptide ou d'un acide aminé, et en présence d'un sel d'ammonium d'au moins un adjuvant de couplage peptidique, le cation ammonium étant sélectionné au sein du groupe constitué par l'ammoniac protoné, une amine primaire protonée et une amine secondaire protonée, le pH étant régulé lors de la réaction pour être compris entre 8 et 9 et la chaîne latérale et la fonction α-amino dudit acide aminé ou peptide étant protégées par des groupements protecteurs résistant aux conditions basiques.

2. Méthode d'amidation du groupement α-carboxy libre d'un acide aminé ou d'un peptide, qui comprend l'étape de réaction dudit acide aminé ou peptide avec un réactif de couplage peptidique dans un solvant organique aprotique en présence d'une première base, à l'exclusion d'une fonction α-amino d'un peptide ou d'un acide aminé, et en présence d'un sel d'ammonium d'au moins un adjuvant de couplage peptidique, le cation ammonium étant sélectionné au sein du groupe constitué par l'ammoniac protoné, une amine primaire protonée et une amine secondaire protonée, le pH étant régulé lors de la réaction pour être compris entre 8 et 9 et la chaîne latérale et la fonction α-amino dudit acide aminé ou peptide étant protégées par des groupements protecteurs résistant aux conditions basiques, à l'exception des groupements ω-carbonyle de résidus aspartyle ou glutamyle individuels, lesdits groupements ω-carbonyle étant amidés en même temps que le groupement α-carboxy libre de la réaction.

3. Méthode selon la revendication 1 ou 2, **caractérisée en ce que** la première base est une base faible dont l'acide conjugué a une valeur de pKa comprise entre 7,5 et 15, préférentiellement entre 7,5 et 10.

4. Méthode selon la revendication 3, **caractérisée en ce que** la base faible est la *N,N* diisopropyléthylamine, une 2,4,6-trialkylpyridine ou une *N*-alkylmorpholine, les groupements alkyles étant préférentiellement des groupements alkyle linéaires ou ramifiés en C₁-C₄.

5. Méthode selon l'une quelconque des revendications précédentes, **caractérisée en ce que** le réactif de couplage est sélectionné au sein du groupe constitué par les sels d'uronium et les sels de phosphonium du benzotriazole susceptibles d'activer ledit groupement α-carboxy.

6. Méthode selon l'une quelconque des revendications précédentes, **caractérisée en ce que** le solvant organique est un solvant organique polaire aprotique.

7. Méthode selon la revendication 6, **caractérisée en ce que** le solvant est sélectionné au sein du groupe constitué par les solvants suivants : acétonitrile, diméthylsulfoxyde, diméthylacétamide, dichlorométhane et *N,N*-diméthylformamide, et **en ce qu'**il s'agit préférentiellement du *N,N*-diméthylformamide.

8. Méthode selon l'une quelconque des revendications précédentes, **caractérisée en ce que** l'adjuvant du réactif de couplage est un dérivé *N*-hydroxy nucléophile susceptible de former des esters activés.

9. Méthode selon la revendication 8, **caractérisée en ce que** l'adjuvant du réactif de couplage est sélectionné au sein du groupe constitué par les composés suivants : *N-*hydroxysuccinimide, *N*-hydroxy-3,4-dihydro-4-oxo-1,2,3-benzotriazine, 1-hydroxy-7-azabenzotriazole et *N*-hydroxybenzotriazole.

10. Méthode selon la revendication 9, **caractérisée en ce que** l'adjuvant du réactif de couplage est l'hydroxy-3,4-dihydro-4-oxo-1,2,3-benzotriazine.

11. Méthode selon l'une quelconque des revendications précédentes, **caractérisée en ce que** le pH est régulé lors de la réaction pour rester compris entre 8 et 9, et **en ce qu'**il est préférentiellement régulé à environ 8,5.

12. Méthode selon les revendications 1 ou 2, **caractérisée en ce qu'**elle est menée en employant un carbodiimide au titre de réactif de couplage et, préférentiellement, en présence d'un *N*-hydroxybenzotriazole protoné au titre de second adjuvant de couplage.

13. Méthode selon la revendication 5, **caractérisée en ce que** le réactif de couplage de type sel d'uronium ou de phosphonium est un sel d'uronium, et **en ce qu'**il s'agit préférentiellement de HCTU, de TCTU ou de HBTU, et plus préférentiellement **en ce qu'**il est employé dans la réaction en combinaison avec un sel d'ammonium de la *N*-hydroxy-3,4-dihydro-4-oxo-1,2,3-benzotriazine.

14. Méthode selon l'une quelconque des revendications précédentes, **caractérisée en ce que** le cation ammonium est H₂N⁺R¹R², R¹ et R² représentant chacun de façon indépendante H ou un hydrocarbure aliphatique ou alicyclique en C₁-C₁₀, préférentiellement en C₁-C₅.

15. Méthode selon la revendication 14, **caractérisée en ce que** R¹ représente H et R² représente un groupement méthyle, éthyle, propyle ou isopropyle, ou **en ce que** R¹ et R² représentent tous deux H.

16. Méthode selon l'une quelconque des revendications précédentes, **caractérisée en ce que** dans une seconde étape après la première étape de la réaction, l'acide aminé ou peptide protégé et amidé est isolé, et **en ce que** dans une troisième étape, il est déprotégé pour obtenir les chaînes latérales libres et la fonction α-amino libre, sachant qu'il est préférentiellement partiellement ou entièrement déprotégé par acidolyse.

17. Méthode d'amidation du groupement α-carboxy libre d'un peptide ILRWPWWPWRRK, qui comprend la première étape de réaction dudit ILRWPWWPWRRK avec un réactif de couplage peptidique dans un solvant organique aprotique en présence d'une première base, à l'exclusion d'une fonction α-amino d'un peptide ou d'un acide aminé, et en présence d'un sel d'ammonium d'au moins un adjuvant de couplage peptidique, le cation ammonium étant sélectionné au sein du groupe constitué par l'ammoniac protoné, une amine primaire protonée et une amine secondaire protonée, le pH étant régulé lors de la réaction pour être compris entre 8 et 9 et la chaîne latérale et la fonction α-amino dudit ILRWPWWPWRRK étant protégées par des groupements protecteurs résistant aux conditions basiques.

18. Méthode d'amidation du groupement α-carboxy libre d'un peptide antimicrobien, qui comprend la première étape de réaction dudit peptide avec un réactif de couplage peptidique dans un solvant organique aprotique en présence d'une première base, à l'exclusion d'une fonction α-amino d'un peptide ou d'un acide aminé, et en présence d'un sel d'ammonium d'au moins un adjuvant de couplage peptidique, le cation ammonium étant sélectionné au sein du groupe constitué par l'ammoniac protoné, une amine primaire protonée et une amine secondaire protonée, le pH étant régulé lors de la réaction pour être compris entre 8 et 9 et la chaîne latérale et la fonction α-amino dudit peptide étant protégées par des groupements protecteurs résistant aux conditions basiques.

19. Méthode selon l'une des revendications 17 ou 18, **caractérisée en ce que** dans une seconde étape après la première étape de la réaction, le peptide ILRWPWWPWRRK ou antimicrobien protégé et amidé, préférentiellement un peptide cationique antimicrobien, est isolé, et **en ce que** dans une troisième étape, il est déprotégé pour obtenir les chaînes latérales libres et la fonction α-amino libre, sachant qu'il est préférentiellement partiellement ou entièrement déprotégé par acidolyse.
